# EUROPÄISCHE PATENTSCHRIFT

(11) **EP 2 850 997 B1**
(45) Veröffentlichungstag und Bekanntmachung des Hinweises auf die Patenterteilung: **02.08.2017**
(21) Anmeldenummer: 14185857.1
(22) Anmeldetag: 22.09.2014
(51) Int. Cl.: A61B 1/00, A61B 1/05, A61B 90/30, A61B 90/00

(54) **Endoskop oder Exoskop**
Endoscope or exoscope
Endoscope ou exoscope

(30) Priorität: 24.09.2013 DE 102013110544
(43) Veröffentlichungstag der Anmeldung: 25.03.2015
(73) Patentinhaber: Karl Storz GmbH & Co. KG, 78532 Tuttlingen (DE)
(72) Erfinder: Heni, Andreas, 78532 Tuttlingen (DE); Kupferschmid, Markus, 78532 Tuttlingen (DE); Ulmschneider, Daniel, 78532 Tuttlingen (DE); Graf, Christian, 78532 Tuttlingen (DE); Schwarz, Peter, 78532 Tuttlingen (DE); Pilz, Kevin, 78532 Tuttlingen (DE)

(56) Entgegenhaltungen:
- WO-A1-2013/051168
- JP-A- S6 084 524
- US-A1- 2008 249 418

## Beschreibung

Die vorliegende Erfindung betrifft ein Endoskop oder ein Exoskop nach dem Oberbegriff von Anspruch 1.

Endoskope werden heute für eine Vielzahl von Anwendungen in Medizin und Technik verwendet. Endoskope umfassen typischerweise einen starren oder flexiblen lang erstreckten Schaft, der zur Einführung in einen Hohlraum geeignet ist, und in dessen distalem (d.h. beobachterfernen) Endbereich ein Objektiv zur Erzeugung eines Bildes eines Objektfelds in dem Hohlraum angeordnet ist. Zur Aufnahme und Weiterleitung des endoskopischen Bildes vom distalen Ende des Endoskops zum proximalen (beobachternahen) Ende können ein elektronischer Bildaufnehmer, beispielsweise ein CCD-Chip, im Bereich des distalen Endes des Schafts sowie elektrische Leitungen innerhalb des Schafts vorgesehen sein. Das vom elektronischen Bildaufnehmer aufgenommene Bild kann von einer am proximalen Ende des Endoskops anschließbaren Auswertungs- und Anzeigeeinrichtung ausgewertet, auf einem Bildschirm für einen Beobachter dargestellt und/oder beispielsweise für Dokumentationszwecke gespeichert werden. Da in der Regel in dem beobachteten Hohlraum nicht ausreichend Licht vorhanden ist, ist ferner innerhalb des Schafts ein Lichtleitsystem vorgesehen, um ausreichend Licht an das distale Ende des Endoskops zu transportieren, wo es zur Beleuchtung des Hohlraums genutzt wird.

Wenn ein Endoskop, das in seinem distalen Endbereich ein Objektiv und einen diesem zugeordneten elektronischen Bildaufnehmer aufweist, um die Längsachse des Schafts gedreht wird, so dreht sich dabei auch das auf dem Bildschirm dargestellte aufgenommene Bild. Hierdurch wird für einen Beobachter, der beispielsweise der Operateur sein kann, der eine chirurgische Operation durchführt, die Orientierung innerhalb des Hohlraums erschwert oder unmöglich gemacht. Dies gilt insbesondere dann, wenn das Endoskop eine Stereooptik zur Erzeugung eines stereoskopischen Bilds aufweist, da in diesem Fall auch eine Rotation der Stereobasis auftritt.

Aus JP 10192233 A ist ein stereoskopisches elektronisches Endoskop mit einer Schrägblickoptik bekannt, wobei eine motorische Verstellung eines Rotationselements vorgesehen ist, das ein optisches System mit zwei quer zur optischen Achse beabstandeten Objektivlinsensystemen und zwei diesen zugeordneten CCD-Bildaufnehmern trägt. Die Signalkabel der CCD-Bildaufnehmer sind in einem flexiblen Schlauch in einer axialen Bohrung des Rotationselements geführt. In US 5,689,365 ist ein stereoskopisches Endoskop offenbart, das ein vorderes optisches System mit einer einzigen optischen Achse und ein rückwärtiges optisches System mit einer Mehrzahl optischer Achsen aufweist. Das rückwärtige optische System kann gemeinsam mit zwei photoelektrischen Bildaufnehmern relativ zum vorderen optischen System um eine zum Schaft des Endoskops parallele Achse gedreht werden. Die Signalleitung der Bildaufnehmer ist in Verlängerung der Drehachse der Stereooptik geführt und verdrillt. Die beschriebenen Anordnungen sind hinsichtlich der Dauerhaltbarkeit der Kabel nicht optimal.

Aus DE 10 2011 054 031 A1 ist eine Vorrichtung zum Beobachten und Beleuchten eines Objektfelds an einem Patienten von einer Stelle abseits des Körpers des Patienten bekannt, die eine Optik zum Beobachten des Objektfelds und eine Beleuchtung zum Beleuchten des Objektfelds aufweist. Die Vorrichtung weist weiterhin einen Schaft auf, an dessen distalem Ende ein gegenüber dem Schaft erweitertes Kopfteil angeordnet ist, in dem zumindest eine ausstrahlende Beleuchtungseinheit zur homogenen Ausleuchtung des Objektfelds angeordnet ist. Im Schaft verlaufen Zuleitungen für die zumindest eine Beleuchtungseinheit. Ferner kann der langerstreckte Schaft einen Bildweiterleiter aufnehmen, der das Bild des Operationsfelds zu einem proximalen Ende des Schafts weiterleitet. Eine derartige Vorrichtung wird als "Exoskop" bezeichnet. Ein solches Exoskop ermöglicht insbesondere die Beleuchtung und Beobachtung eines Operationsfelds bei einer chirurgischen Operation aus einem Arbeitsabstand von beispielsweise 25 bis 75 cm, so dass der Arbeitsraum des Operateurs durch das Exoskop praktisch nicht eingeschränkt wird. Hierfür kann das Exoskop an einer Halterung in einer geeigneten Position und Orientierung fixiert werden. Auch bei einem Exoskop stellt sich das oben erwähnte Problem, dass sich das von einem elektronischen Bildaufnehmer aufgenommene Bild bei einer Drehung des Exoskops auf dem Bildschirm, auf dem es für einen Beobachter angezeigt wird, dreht.

Die WO2013/051168A1 offenbart ein Endoskop mit einem Schaft und einer drehbar aufgenommenen Bildaufnahmeeinheit, die an ein Kabel angeschlossen ist, das bei Drehung der Bildaufnahmeeinheit teilweise auf diese aufwickelbar ist.

Die US2008/0249418A1 offenbart eine Ultraschallsonde mit einem Kopf, in dem Ultraschallwandler in einer Einheit drehbar aufgenommen sind. Die drehbare Einheit ist an ein Kabel angeschlossen, das bei Drehung der Einheit auf diese aufgewickelt werden kann.

Die JPS6084524A zeigt ein Instrument mit variabler Blickrichtung. Das Instrument umfasst ein schwenkbar gelagertes Objektiv, das an ein Kabel angeschlossen ist, welches sich bei Verschwenken des Objektivs wenigstens teilweise auf das Objektiv aufwickelt.

Es ist Aufgabe der vorliegenden Erfindung, ein gattungsgemäßes Endoskop oder Exoskop mit einem drehbar angeordneten elektronischen Bildaufnehmer und einer verbesserten Kabelführung anzugeben.

Diese Aufgabe wird durch eine Vorrichtung gemäß Anspruch 1 gelöst. Vorteilhafte Weiterbildungen der Erfindung ergeben sich aus den Unteransprüchen.

Ein erfindungsgemäßes Endoskop oder Exoskop weist einen Schaft auf, der vorzugsweise lang erstreckt und starr ausgebildet ist, jedoch auch sehr kurz ausgebildet sein kann, und weist zum Aufnehmen eines Bilds eines Objektfelds mindestens ein Objektiv und mindestens einen elektronischen Bildaufnehmer auf, wobei das mindestens eine Objektiv und der mindestens eine elektronische Bildaufnehmer in einem distalen Endbereich des Endoskops oder Exoskops angeordnet sind. Der distale Endbereich des Endoskops oder Exoskops kann ein distaler Endabschnitt des Schafts sein oder auch ein gegenüber dem Schaft erweitertes Kopfteil, das an einem distalen Ende des Schafts angeordnet ist. Weiter umfasst das Endoskop oder Exoskop mindestens ein Kabel zur Versorgung und/oder Signalübertragung des bzw. von dem mindestens einen elektronischen Bildaufnehmer, das innerhalb des Endoskops oder Exoskops geführt ist. Insbesondere kann das Kabel innerhalb des Schafts geführt sein und von einem proximalen Endabschnitt des Schafts bis zu einem distalen Endabschnitt des Schafts bzw. zu einem am distalen Ende des Schafts angeordneten Kopfteil verlaufen und mit dem dort angeordneten mindestens einen elektronischen Bildaufnehmer verbunden sein. Am proximalen Endabschnitt des Schafts oder auch an einem mit dem proximalen Endabschnitt des Schafts verbundenen Handgriff können Anschlüsse zur Vermittlung einer Verbindung zu externen Versorgungs-, Auswertungs- und/oder Anzeigeeinrichtungen vorgesehen sein. Das Kabel dient insbesondere der Ansteuerung des mindestens einen elektronischen Bildaufnehmers und dessen Versorgung mit elektrischer Energie sowie der Signalübertragung von dem mindestens einen elektronischen Bildaufnehmer zu einer externen Auswertungs- und Anzeigeeinrichtung zur Auswertung und Darstellung der aufgenommenen Bildsignale. Das Kabel kann auch nebeneinander verlaufende, mechanisch voneinander getrennte Teile umfassen. Vorzugsweise ist das Kabel als Flachbandkabel oder als Flexplatine ausgebildet.

Erfindungsgemäß weist das Endoskop oder Exoskop eine um eine erste Drehachse drehbare Optikeinheit auf, die den mindestens einen elektronischen Bildaufnehmer umfasst, der somit um die erste Drehachse drehbar gelagert ist. Die Drehachse ist identisch mit oder näherungsweise parallel zur Blickrichtung des Endoskops oder Exoskops. An der Optikeinheit ist das Kabel angeschlossen und auf einer Umfangsfläche der Optikeinheit zumindest über einen begrenzten Winkelbereich aufwickelbar. Die Optikeinheit umfasst insbesondere auch das mindestens eine Objektiv, dem der elektronische Bildaufnehmer zum Aufnehmen des Bilds des Objektfelds zugeordnet ist, sowie gegebenenfalls weitere mechanische, optische und/oder elektronische Bauelemente. Die Blickrichtung kann beispielsweise durch die optische Achse des mindestens einen Objektivs definiert sein. Die Umfangsfläche der Optikeinheit kann insbesondere eine zylindrische Mantelfläche sein, innerhalb derer der elektronische Bildaufnehmer sowie das Objektiv und ggf. weitere Bauelemente angeordnet sind. Ferner ist erfindungsgemäß vorgesehen, dass der distale Endbereich des Endoskops oder Exoskops, d.h. insbesondere der distale Endabschnitt des Schafts bzw. das gegenüber dem Schaft erweiterte Kopfteil, einen inneren Hohlraum aufweist, in dem ein Kabelreservoir aufgenommen ist, das einen bei einer Drehung der Optikeinheit aufgewickelten Kabelabschnitt bereitstellt und einen bei einer Drehung der Optikeinheit in einer umgekehrten Drehrichtung abgewickelten Kabelabschnitt aufnimmt.

Dabei wird sich das distale Ende des Kabels, welches an der Optikeinheit angeschlossen ist, mit der Drehung der Optikeinheit bewegen. Ein distaler Abschnitt des Kabels wird beim Abwickeln insbesondere in den Hohlraum geschoben. Das proximale Ende des Kabels im Instrument ist dabei in axialer Richtung fest oder annähernd fest gelagert.

Das Endoskop oder Exoskop kann beispielsweise auch eine Stereooptik sein. Der mindestens eine elektronische Bildaufnehmer kann beispielsweise ein CCD (charge-coupled device) sein. Eine solche Stereooptik umfasst insbesondere eine Mehrzahl an Objektiven, die in einer Richtung quer zu einer Blickrichtung der Beobachtungsoptik beabstandet sind, und die jeweils ein Bild des Objektfelds auf jeweils einem oder auch einem gemeinsam zugeordneten elektronischen Bildaufnehmer erzeugen. Die Objektive und Bildaufnehmer sind innerhalb der Optikeinheit angeordnet. Insbesondere sind die Objektive in einer festen räumlichen Beziehung zueinander angeordnet, wobei der quer zur Blickrichtung gemessene Abstand zwischen zwei Objektiven die Stereobasis darstellt. Die Objektive sind mit ihren jeweiligen optischen Achsen parallel oder in einem Winkel zueinander, der durch die Stereobasis und den gewünschten Arbeitsabstand bestimmt ist, angeordnet. Durch eine winklige Anordnung kann es erreicht werden, dass bei dem bevorzugten Arbeitsabstand die Mitte jedes Halbbilds des stereoskopischen Bilds denselben Punkt des Objektfelds darstellt. Als "Blickrichtung" des Endoskops oder Exoskops wird in dem Fall, dass die optischen Achsen der Objektive in einem Winkel zueinander stehen, eine zwischen den optischen Achsen stehende mittlere Richtung bezeichnet. Die Optikeinheit umfasst dann beispielsweise zwei Bildsensoren und / oder zwei Objektive zur Erzeugung des Stereobilds.

Endoskope und Exoskope weisen distale Beleuchtungseinheiten auf, um eine zu betrachtende Stelle ausreichend auszuleuchten. Diese können distal angebrachte Beleuchtungseinheiten wie LEDs sein. Es können auch Lichtleiter, insbesondere mit distal angeschlossenem Fenster oder optischen Elementen als Beleuchtungseinheit vorgesehen sein.

Dadurch, dass eine drehbare Optikeinheit vorgesehen ist, die den elektronischen Bildaufnehmer umfasst und auf deren Umfangsfläche das Versorgungs- und/oder Signalkabel des elektronischen Bildaufnehmers auf- bzw. abwickelbar ist, und dass im distalen Endbereich des Endoskops oder Exoskops ein Hohlraum vorgesehen ist, in dem ein Kabelreservoir angeordnet ist, wird es auf einfache Weise ermöglicht, den mit der Optikeinheit drehbar gelagerten elektronischen Bildaufnehmer mit elektrischer Energie zu versorgen bzw. Signale zum und vom elektronischen Bildaufnehmer zu übertragen. Eine Verdrillung des Kabels ist dabei nicht notwendig. Ebenso kann eine Verschiebung des Kabels innerhalb des Schafts, womit ein Verschleiß des Kabels und ggf. weiterer Bauteile verbunden wäre, vermieden werden. Insbesondere kann das Kabel innerhalb des Schafts fest verlegt sein. Dabei ist gleichzeitig sichergestellt, dass das Kabel die Verwendung des Endoskops bzw. Exoskops nicht beeinträchtigt und insbesondere keine Einschränkung des Gesichtsfelds des Endoskops oder Exoskops bzw. des Arbeitsbereichs eines Operateurs bei einem chirurgischen Eingriff, der unter Beobachtung durch das Endoskop bzw. Exoskop durchgeführt wird, darstellt. Schließlich kann auf diese Weise auch erreicht werden, dass das Kabel weitgehend vor Verschmutzung bei einem solchen chirurgischen Eingriff geschützt ist und das Endoskop bzw. Exoskop auf einfache Weise reinigbar und sterilisierbar ist.

Insbesondere kann durch die erfindungsgemäße Konstruktion eine Drehung der Optikeinheit um 180° ohne Beschädigung des angeschlossenen Kabels dauerhaft ermöglicht werden.

Gemäß einer bevorzugten Ausführungsform der Erfindung weist der Endbereich des Endoskops oder Exoskops ein hermetisch dichtes Gehäuse auf, innerhalb dessen der Hohlraum gebildet ist, in dem das Kabelreservoir aufgenommen ist. Innerhalb des hermetisch dichten Gehäuses ist die Optikeinheit mit dem mindestens einem elektronischen Bildaufnehmer aufgenommen und drehbar gelagert. Insbesondere kann das hermetisch dichte Gehäuse autoklavfest ausgebildet sein, wodurch eine besonders einfache Reinigung und Sterilisierung des Endoskops bzw. Exoskops ermöglicht wird. Durch die erfindungsgemäße Ausgestaltung kann dabei der besondere Vorteil erzielt werden, dass das Kabel beim Durchtritt durch das hermetisch dichte Gehäuse, beispielsweise aus einem Kopfteil in den Schaft des Endoskops bzw. Exoskops, bei einer Drehung der Optikeinheit keine Verschiebung oder sonstige Bewegung ausführt und somit ein hermetisch dichter Durchtritt des Kabels durch das Gehäuse auf einfache Weise erreichbar ist.

Vorzugsweise ist die erste Drehachse im Wesentlichen senkrecht zu einer Längsachse des Schafts gerichtet. In besonders vorteilhafter Weise ist das Endoskop bzw. Exoskop mit einer 90°-Optik ausgebildet, d.h. eine Blickrichtung des Endoskops oder Exoskops steht in einem Winkel von näherungsweise 90° zur Längsachse des Schafts; in diesem Fall ist die erste Drehachse parallel zur Blickrichtung des Endoskops oder Exoskops. Eine derartige Bauart ermöglicht insbesondere bei einem Exoskop eine Beobachtung eines Operationsfelds ohne wesentliche Einschränkung des Arbeitsraums des Operateurs und mit einer vergleichsweise einfach aufgebauten Halterung.

Gemäß einer bevorzugten Ausführungsform der Erfindung steht die erste Drehachse im Wesentlichen senkrecht zu einer Längsachse des Schafts, und das Auf- bzw. Abwickeln des Kabels auf die bzw. von der Optikeinheit erfolgt in einer im Wesentlichen tangentialen Richtung, die im Wesentlichen parallel zur Längsachse des Schafts steht. Hierdurch wird erreicht, dass die Aufnahme eines abgewickelten bzw. das Zuführen eines aufzuwickelnden Kabelabschnitts in Schaftrichtung erfolgt, so dass das Kabelreservoir von der Optikeinheit aus gesehen ebenfalls im Wesentlichen in Schaftrichtung angeordnet sein kann. Ferner kann das Kabelreservoir in vorteilhafter Weise derart ausgebildet sein, dass es eine größere Erstreckung in Längsrichtung des Schafts als in Querrichtung aufweist. Hierdurch wird eine besonders kompakte und platzsparende Bauweise ermöglicht.

Gemäß einer weiteren bevorzugten Ausführungsform ist das Kabel in einem vollständig abgewickelten Zustand, in Richtung der Längsachse des Schafts betrachtet, seitlich an der Optikeinheit angeschlossen.

Dadurch, dass das Kabel nicht etwa an einer proximalen Stelle der Optikeinheit mit dieser verbunden ist, sondern seitlich an der Optikeinheit, wird erreicht, dass sich das Kabel definiert in den Hohlraum legen kann. Es wird insbesondere auch nicht beim Drehen der Optikeinheit geknickt, sondern lediglich der abgewickelte Teil des Kabels definiert in den Hohlraum hinein geschoben. Dadurch wird das Kabel besonders geschont und eine übermäßige Belastung oder eine Ermüdung des Materials vermieden.

Erfindungsgemäß ist das Kabel innerhalb des Hohlraums in Form mindestens eines Bogens gelegt; ein solcher Bogen kann auch als "Bucht" bezeichnet werden. Der den Bogen bildende Kabelabschnitt ist ein von der Umfangsfläche der Optikeinheit bei einer Drehung der Optikeinheit in einer entsprechenden Drehrichtung abgewickelter Kabelabschnitt oder ein sich an diesen anschließender. Eine Länge des Kabels, die etwa der von dem Bogen aufgenommenen Länge entspricht, steht zum Aufwickeln auf die Umfangsfläche der Optikeinheit zur Verfügung. Es kann auch vorgesehen sein, dass der Kabelabschnitt innerhalb des Hohlraums mäanderförmig gelegt wird, d.h. dass mehr als ein Bogen vorgesehen ist, wobei die aufeinander folgenden Bögen gegenläufige Krümmungen haben. Ferner können weitere, gegebenenfalls feststehende und somit von der Drehung der Optikeinheit unabhängige Biegungen bzw. Umlenkungen des Kabels vorgesehen sein. Das Kabel kann zur Sicherstellung, dass es sich in Form des mindestens einen Bogens in den Hohlraum legt, elastisch vorgekrümmt bzw. selbst als entsprechend geformte Feder ausgebildet sein; dies kann beispielsweise durch eine Elastomer-Beschichtung, die im gekrümmten Zustand eines Flachbandkabels bzw. einer Flexplatine aufgebracht wird, erreicht werden. Dadurch, dass innerhalb des Kabelreservoirs das Kabel mindestens einen Bogen bildet, ist auf einfache Weise die Aufnahme bzw. Bereitstellung eines ab- bzw. aufgewickelten Kabelabschnitts möglich, ohne dass das Kabel beschädigt wird

Gemäß einer nicht beanspruchten Ausführungsform ist das Kabel in dem Bereich, in dem es den mindestens einen Bogen bzw. die mindestens eine Bucht bildet, frei in den Hohlraum gelegt, d.h. es ist keine Führung im gekrümmten Bereich des Bogens vorgesehen, allenfalls eine einseitige Anlagefläche für den näherungsweise geraden Teil des Kabels. Dabei kann durch Wahl einer entsprechenden Steifigkeit des Kabels erreicht werden, dass auch ohne eine Führung eine reproduzierbare und verschleißarme Aufnahme bzw. Bereitstellung eines auf- bzw. abgewickelten Kabelabschnitts ermöglicht wird. Hierdurch kann auf besonders einfache Weise eine reibungs- und verschleißarme Kabelverbindung zu der drehbaren Optikeinheit geschaffen werden.

Bevorzugterweise behält der mindestens eine Bogen seine Krümmungsrichtung beim Drehen der Optikeinheit und damit beim Auf- und Abwickeln bei. Lediglich der Radius des Bogens, die Zahl der Bögen oder die Anordnung der Bögen verändern sich, wenn das Kabel durch die Drehung in den Hohlraum geschoben oder wieder herausgezogen wird. Insbesondere wird bei wechselnder Drehrichtung das Kabel nicht wiederholt entgegengesetzten Krümmungen oder Knicken ausgesetzt, die an der gebogenen Stelle zu einer Materialermüdung führen würden. Das Kabel wird hierdurch definiert in dem Hohlraum geführt.

Erfindungsgemäß wird der von der Umfangsfläche der Optikeinheit abgewickelte bzw. auf diese aufzuwickelnde Kabelabschnitt durch mindestens eine in dem Hohlraum angeordnete Rolle geführt. Diese ist vorzugsweise um eine zweite Drehachse drehbar ausgebildet, wobei die zweite Drehachse insbesondere im Wesentlichen parallel zur ersten Drehachse und damit vorzugsweise senkrecht zur Längsachse des Schafts steht. Hierdurch wird eine besonders sichere und gegen Lageänderungen und Erschütterungen des Endoskops bzw. Exoskops unempfindliche Aufnahme und Bereitstellung des Kabelabschnitts ermöglicht. Die mindestens eine Rolle kann auch feststehend, also als nicht drehbare Umlenkung ausgebildet sein. Das Kabel wird über die Außenfläche der Rolle geführt. Die Rolle befindet sich also insbesondere an der Stelle des Kabels, an der dieses einen Bogen bildet.

In einer weiteren Ausgestaltung der Erfindung wird die mindestens eine Rolle durch eine Beleuchtungseinheit gebildet.

Dadurch, dass die Rolle durch eine Beleuchtungseinheit gebildet wird, ist die erfindungsgemäße Kabelführung besonders platzsparend möglich, da ein bereits vorhandenes Element die Funktion der Rolle übernimmt.

Vorzugsweise ist die mindestens eine Rolle, über die der Kabelabschnitt geführt ist, verschiebbar gelagert. Insbesondere ist die Rolle derart gelagert, dass sie in einer zur Längsrichtung des Schafts im Wesentlichen parallelen Richtung verschiebbar ist. Hierdurch wird es ermöglicht, dass das Kabel stets, auch im nicht vollständig auf bzw. abgewickelten Zustand, durch die Rolle geführt ist. Dadurch kann eine weiter verbesserte Sicherheit der Bereitstellung und Aufnahme des Kabelabschnitts erreicht werden.
stand, durch die Rolle geführt ist. Dadurch kann eine weiter verbesserte Sicherheit der Bereitstellung und Aufnahme des Kabelabschnitts erreicht werden.

In besonders vorteilhafter Weise ist die mindestens eine Rolle, über die das Kabel geführt ist, federbelastet verschiebbar. Durch die Federbelastung der Verschiebung der Rolle kann erreicht werden, dass das Kabel stets gespannt ist, wodurch eine weiter verbesserte Sicherheit bei der Führung des Kabels durch die Rolle erreichbar ist. Ein Ausgleich der durch die Federbelastung der Rolle ausgeübten, tangential auf die Optikeinheit einwirkenden Zugkraft kann beispielsweise durch eine entgegenwirkende Feder in der Lagerung der Optikeinheit oder in einem Antrieb der Optikeinheit erreicht werden.

Gemäß einer weiteren bevorzugten Ausführungsform ist das Kabel innerhalb des Hohlraums über zwei Rollen geführt, die auf einem zweiarmigen Hebel gelagert sind, der um eine dritte Achse schwenkbar ist, wobei die dritte Achse im Wesentlichen parallel zur Drehachse der Optikeinheit steht. Insbesondere kann der zweiarmige Hebel federbelastet sein, so dass das Kabel stets sicher an den beiden Rollen anliegt. Dadurch, dass das Kabel auf zwei Rollen, die auf einem zweiarmigen Hebel gelagert sind, geführt wird, kann eine reibungsarme und sichere Nachstellung der Rollen und somit eine besonders sichere Führung des Kabels erreicht werden.

Gemäß einer bevorzugten Ausführungsform ist das Kabelreservoir in Form von zwei in Richtung der ersten Drehachse übereinander angeordneten Schichten ausgebildet, in denen jeweils ein Teil des Kabels oder auch ein weiteres flexibles Element, etwa ein Draht, das ebenfalls auf der Optikeinheit auf- bzw. abgewickelt wird, aufgenommen ist. Dabei sind die in der ersten und in der zweiten Schicht aufgenommenen Abschnitte des Kabels bzw. des flexiblen Elements in entgegengesetzten Richtungen auf der Optikeinheit aufgewickelt. Wenn das Kabel bzw. der in der ersten Schicht aufgenommene Teil des Kabels durch Drehung der Optikeinheit in einer gegebenen Richtung aufgewickelt wird, wird somit der in der zweiten Schicht aufgenommene Teil des Kabels bzw. das in dieser aufgenommene flexible Element gleichzeitig von der Umfangsfläche der Optikeinheit abgewickelt und umgekehrt. Ferner werden hierbei die erste und die zweite Schicht innerhalb des Hohlraums gegenläufig gelegt bzw. geführt, insbesondere gegengleich gelegt bzw. geführt. Hierdurch kann der Vorteil erreicht werden, dass eine durch eine Federung einer Rolle in der ersten Schicht ausgeübte Zugkraft durch eine gegenläufige bzw. gegengleiche Zugkraft der zweiten Schicht kompensiert wird, so dass auf die Optikeinheit insgesamt ein verringertes oder kein Drehmoment ausgeübt wird. Das gleiche gilt für etwaige elastische Kräfte, die durch die Biegung des Kabels entstehen und gegebenenfalls ebenfalls zu einem auf die Optikeinheit wirkenden Drehmoment führen können.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:
Fig. 1a und 1b ein Ausführungsbeispiel eines erfindungsgemäßen Exoskops in zwei unterschiedlichen perspektivischen Ansichten;
Fig. 2 einen senkrechten Längsschnitt durch das Kopfteil des in den Figuren 1a und 1b dargestellten Exoskops;
Fig. 3 eine Seitenansicht des in den Figuren 1a und 1b dargestellten Exoskops;
Fig. 4a bis 4c einen waagrechten Längsschnitt durch das Kopfteil des in den Figuren 1a und 1b dargestellten Exoskops in drei verschiedenen Drehpositionen der Optikeinheit mit einem Kabelreservoir gemäß einer ersten Ausführungsform;
Fig. 5 einen Längsschnitt gemäß Fig. 4a bis 4c mit zusätzlichen Details;
Fig. 6 eine zweite Ausführungsform eines Kabelreservoirs in schematischer Darstellung;
Fig. 7 eine dritte Ausführungsform eines Kabelreservoirs in schematischer Darstellung.

Die Erfindung wird im Folgenden anhand einer Ausführungsform, die als Exoskop ausgebildet ist, erläutert.

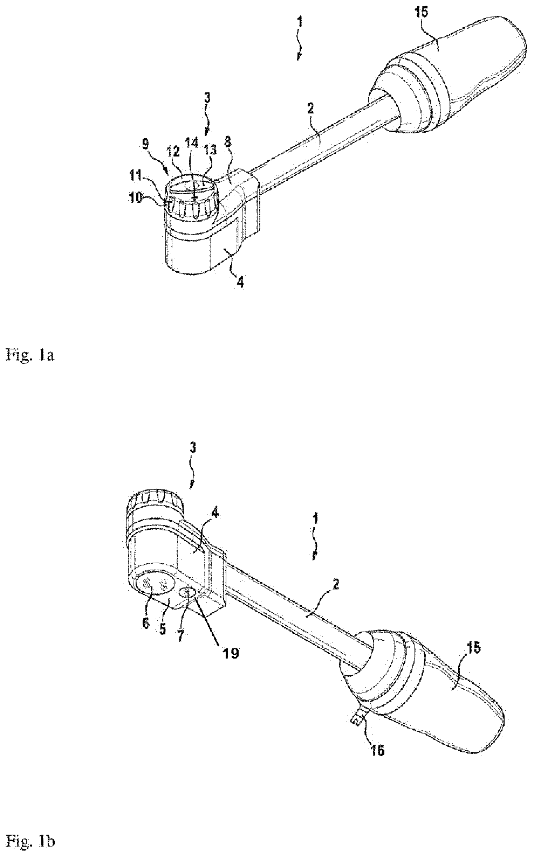

In den Figuren 1a und 1b ist ein solches Exoskop 1 in zwei unterschiedlichen perspektivischen Ansichten gezeigt. Das Exoskop 1 weist einen lang erstreckten zylindrischen Schaft 2 auf, an dessen distalem Ende ein im Vergleich zum Schaft erweitertes Kopfteil 3 angeordnet ist. Das Kopfteil 3 weist ein Gehäuse 4 auf, dessen distaler Bereich näherungsweise halbzylindrisch geformt ist, wobei die Achse des Halbzylinders zur Längsachse des Schafts 2 in einem Winkel von etwa 90° steht. In eine Bodenfläche 5 des Gehäuses ist ein Beobachtungsfenster 6 eingesetzt, durch das eine im Inneren des Gehäuses 4 angeordnete Beobachtungsoptik Licht von einem Objektfeld erhält. Die Bodenfläche 5 weist ferner ein Beleuchtungsfenster 7 auf, hinter bzw. in dem eine Beleuchtungseinheit 19 endet und durch das Beleuchtungslicht zum Beleuchten des Objektfelds abgestrahlt werden kann. Die Beleuchtungseinheit kann beispielsweise eine hinter dem Beleuchtungsfenster 7 im Inneren des Kopfteils angeordnete Lichtquelle sein. Es können aber auch Lichtleiter im Bereich des Beleuchtungsfensters 7 enden, oder es können die Endflächen der Lichtleiter selbst das Beleuchtungsfenster 7 bilden, wobei die Lichtleiter durch den Schaft 2 geführt sind und Licht von einer außerhalb des Kopfteils 3 angeordneten Lichtquelle übertragen. Das Beobachtungsfenster 6 ist vorzugsweise eine planparallele Platte, während das Beleuchtungsfenster 7 vorzugsweise als Konvexlinse ausgebildet ist.

Auf einen distalen Endbereich einer der Bodenfläche 5 gegenüberliegenden Deckelfläche 8 ist eine Drehkappe 9 aufgesetzt, die an ihrer Umfangsseite 10 eine Mehrzahl von Griffmulden 11 aufweist und auf ihrer Oberseite 12 einen Drehgriff 13 trägt. Ferner weist die Drehkappe 9 eine Markierung 14 auf, die die Drehstellung der Drehkappe 9 leicht erkennbar macht. Das Exoskop 1 weist ferner einen Handgriff 15 auf, der einen Anschluss 16 zum Anschließen von Signal- und Versorgungskabeln aufweist. Der Handgriff 15 kann auch beispielsweise eine Kühleinrichtung oder eine Lichtquelle zur Erzeugung von Beleuchtungslicht, das durch Lichtleiter zum Beleuchtungsfenster 7 geleitet wird, enthalten.

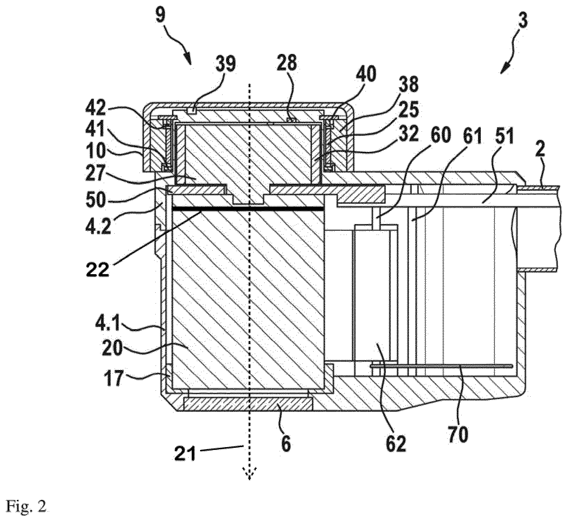

Wie in der Schnittdarstellung der Fig. 2 gezeigt ist, ist das Gehäuse 4 aus einem Gehäuseunterteil 4.1 und einem Gehäuseoberteil 4.2 zusammengesetzt, die hermetisch dicht, beispielsweise durch Schweißen, miteinander verbunden sind. Das Beobachtungsfenster 6 ist hermetisch dicht in die von dem Gehäuseunterteil 4.1 gebildete Bodenfläche 5 eingesetzt, etwa durch Löten, ebenso wie das in Fig. 2 nicht dargestellte Beleuchtungsfenster 7 (s. Fig. 1b). Das Gehäuse 4 ist somit insgesamt selbst hermetisch dicht abgeschlossen oder hermetisch dicht mit dem Schaft 2 des Exoskops verbunden, der in seinem proximalen Bereich hermetisch abgeschlossen ist. Im Inneren des Gehäuses 4 ist eine Optikeinheit 20 angeordnet, die mindestens ein Objektiv mit einem zugeordneten elektronischen Bildaufnehmer 22 sowie ggf. weitere optische und elektronische Bauteile enthält (nicht dargestellt). Das Objektiv definiert eine Blickrichtung 21 des Exoskops, die im dargestellten Beispiel senkrecht zum Fenster 6 und zur Längsachse des Schafts 2 verläuft.

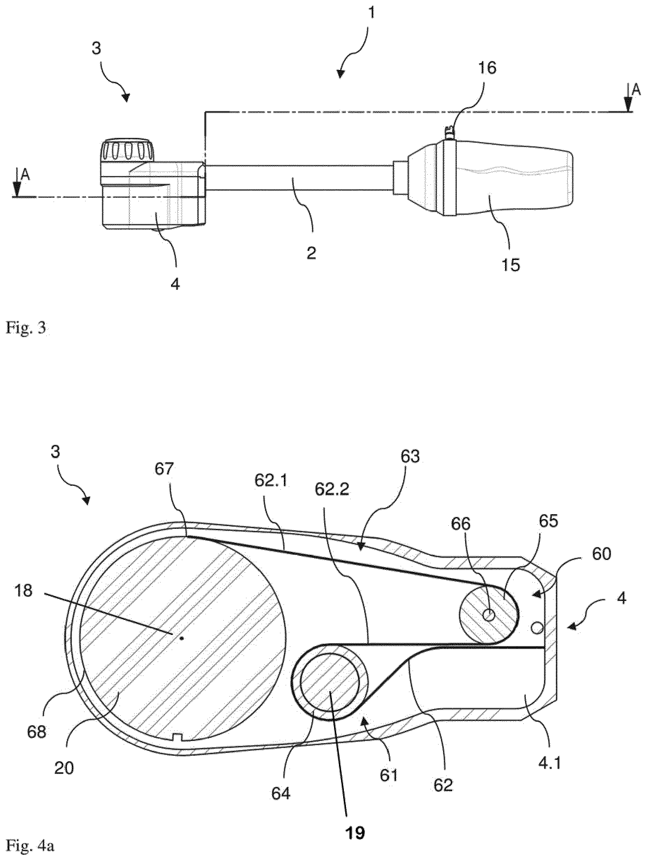

Die Optikeinheit 20 ist innerhalb des Gehäuses 4 mit einem Gleitlager 17 drehbar gelagert und durch einen Magnettrieb von außerhalb des Gehäuses mit einer Drehkappe 9, die auf die dem Beobachtungsfenster 6 entgegengesetzte Seite des Kopfteils 3 aufgesetzt ist, manuell verstellbar. Hierfür weist der Magnettrieb erste Magneten 25 auf, die an der Innenseite eines ringförmigen ersten Magnetträgers 38, der drehfest mit der Drehkappe 9 verbunden ist, befestigt sind, sowie zweite Magneten 32, die an der Mantelfläche eines mit der Optikeinheit 20 drehfest verbundenen, zylindrischen zweiten Magnetträgers 27 gehalten sind. Die ersten und die zweiten Magneten 25, 32 sind derart angeordnet, dass sie durch eine Wand des Gehäuseoberteils 4.2 hindurch zur Übertragung eines Drehmoments vom ersten Magnetträger 38 zum zweiten Magnetträger 27 zusammenwirken. Die Drehkappe 9 mit dem ersten Magnetträger 38 ist um die gleiche Drehachse drehbar wie die Optikeinheit 20 mit dem zweiten Magnetträger 27. Durch eine Drehung der Drehkappe 9 kann somit eine entsprechende Drehung der Optikeinheit bewirkt werden. Der mögliche Verstellwinkel der Drehkappe 9 wird durch einen Anschlag 39 begrenzt, während der Umdrehungswinkel der Optikeinheit 20 durch einen Anschlag 28 begrenzt wird. Hierdurch wird neben der Begrenzung der Winkel auch die Sicherstellung einer optimalen Kopplung zwischen den magnetischen Koppelelementen ermöglicht. Als Verstellwinkelbereich ist in der Regel ein Bereich von ± 90° ausreichend. Zur Abdichtung der Drehkappe 9 sind Gleitdichtringe 40, 41 vorgesehen. Die Optikeinheit 20 ist mit einer im Wesentlichen zylindrischen Mantel- bzw. Umfangsfläche ausgebildet, wobei die Drehachse 18 mit der Zylinderachse und der Blickrichtung 21 übereinstimmt (s. Fig. 4a).

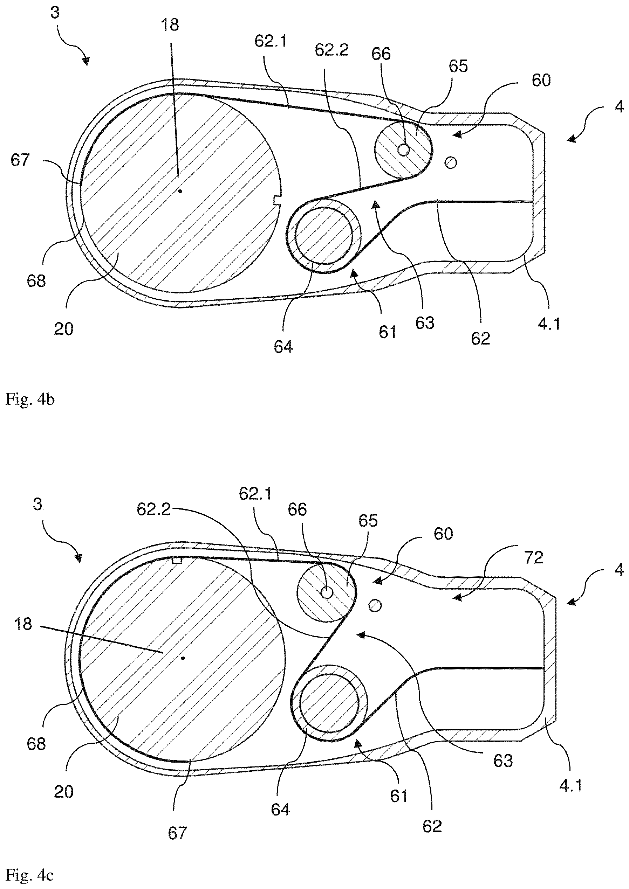

In Fig. 2 sind exemplarisch Maßnahmen zur Wärmeabführung dargestellt. Auf der dem Gleitlager 17 gegenüberliegenden Seite ist die Optikeinheit 20 mit Wärmeleitblechen 50 aus Graphit in Kontakt, durch die in der Optikeinheit 20 erzeugte Verlustwärme in eine Heatpipe 51 weitergeleitet wird, wodurch die Verlustwärme durch den Schaft 2 in den proximalen Endbereich des Exoskops 1 bzw. in den Griff 15 abgeführt wird. Ferner ist in Fig. 4 schematisch eine Führung von Verbindungsleitungen zur Optikeinheit 20 angedeutet. Hierfür sind Rollen in Form von Umlenkungen 60, 61 vorgesehen, über die ein Kabel 62, das beispielsweise als Flachbandkabel oder als Flexplatine ausgebildet sein kann, geführt wird. Zur Verschiebung der Umlenkung 60 ist eine Führung 70 vorgesehen. Das hierdurch gebildete Kabelreservoir, das den bei einer Drehung der Optikeinheit 20 auf die Umfangsfläche der Optikeinheit 20 aufzuwickelnden Abschnitt des Kabels 62 zur Verfügung stellt bzw. den bei einer entgegengesetzten Drehung abgewickelten Abschnitt aufnimmt, wird im Folgenden näher erläutert.

In Fig. 3 ist in einer Seitenansicht das zuvor beschriebene Exoskop 1 gezeigt, wozu auf die Beschreibung zu den Figuren 1a und 1b verwiesen wird. In Figur 3 ist die Schnittlinie A-A eingetragen, entlang derer der in den Figuren 4a bis 4c dargestellte waagrechte Längsschnitt durch das Kopfteil geschnitten ist.

Die Figuren 4a bis 4c zeigen das Kabelreservoir gemäß einer ersten Ausführungsform in einem waagrechten Längsschnitt in unterschiedlichen Drehstellungen der Optikeinheit 20, die um die Drehachse 18 drehbar gelagert ist, wobei Fig. 4a und 4c die einer Drehung um ca. 180° entsprechenden Endstellungen und Fig. 4b eine dazwischenliegende Drehstellung zeigen. Der Umdrehungswinkel der Optikeinheit 20 ist durch einen oder mehrere Anschläge begrenzt.

Wie in Figur 4a zu erkennen ist, sind innerhalb des hermetisch dichten Gehäuses 4, dessen Gehäuseunterteil 4.1 in Figur A geschnitten ist, die im Wesentlichen zylindrisch geformte Optikeinheit 20 und das Kabelreservoir 63 aufgenommen, das einen Abschnitt des Kabels 62 sowie die als Rollen dienenden Umlenkungen 60, 61 umfasst. Die Umlenkung 61 umfasst einen feststehenden Zylinder 64, der fest in dem Gehäuse 4 gehalten ist. Der feststehende Zylinder 64 enthält eine Beleuchtungseinheit 19, beispielsweise in Form eines distalen Endabschnitts der Lichtleiter zur Beleuchtung des Objektfelds, wodurch eine besonders platzsparende Bauweise ermöglicht wird. Die weitere Umlenkung 60 umfasst eine zylindrische Rolle 65, die um eine Drehachse 66 drehbar gelagert ist. In der in Figur 4a gezeigten Drehstellung der im Wesentlichen zylindrisch ausgebildeten Optikeinheit 20 setzt das Kabel in einem Anschlusspunkt 67 an der Umfangsfläche 68 der Optikeinheit an, von wo es ins Innere der Optikeinheit zum Anschluss an den elektronischen Bildaufnehmer sowie gegebenenfalls weitere elektronische Bauelemente geführt ist, ist jedoch nicht auf die Umfangsfläche 68 der Optikeinheit aufgewickelt. Dementsprechend nimmt das Kabelreservoir einen relativ großen Abschnitt des Kabels auf, wofür die Umlenkung 60 in einer proximalen Endstellung angeordnet ist. In diesem vollständig abgewickelten Zustand des Kabels, liegt sein Anschlusspunkt 67 an die Optikeinheit 20 in Längsrichtung des Schafts betrachtet, d.h. wie dargestellt entlang des Schnitts A-A in Figur 3, seitlich an der Optikeinheit 20.

In der in Figur 4b gezeigten Drehstellung der Optikeinheit 20 ist das Kabel 62 über einen Winkelbereich von ca. 90° auf die Umfangsfläche 68 der Optikeinheit aufgewickelt. Das Kabelreservoir 63 muss daher nur noch einen kürzeren Abschnitt des Kabels 62 aufnehmen. Dementsprechend befindet sich die Umlenkung 60 in einer mittleren Position, wie in Figur 4b schematisch dargestellt ist.

In der in Figur 4c gezeigten Situation ist das Kabel 62 auf einen etwa 180° umfassenden Bereich der Umfangsfläche 68 der Optikeinheit 20 aufgewickelt. Das Kabelreservoir 63 hat in dieser Situation den verfügbaren Abschnitt des Kabels 62 praktisch vollständig zur Verfügung gestellt und umfasst nur noch einen Mindestabschnitt des Kabels 62, der zur Aufrechterhaltung einer sicheren Führung erforderlich ist. Die aufgewickelte und vom Kabelreservoir 63 zur Verfügung zu stellende Länge des Kabels 62, die beim Abwickeln vom Kabelreservoir 63 wieder aufzunehmen ist, beträgt bei einem Durchmesser der zylindrischen Umfangsfläche von beispielsweise 35 mm etwa 55 mm.

In einer alternativen, nicht dargestellten Ausführungsform sind keine Umlenkungen 60, 61 vorgesehen. Stattdessen wird das Kabel frei im Hohlraum geführt. Der Verlauf des Kabels entspricht dann dem in den Figuren 4a-4c. Das Kabel legt sich in diesem Fall in zwei Bögen, deren Radius und Position sich beim Drehen der Optikeinheit verändern, deren Krümmungsrichtung jedoch gleich bleibt. Auf diese Weise ist wird ein wechselndes Umbiegen oder Knicken des Kabels vermieden.

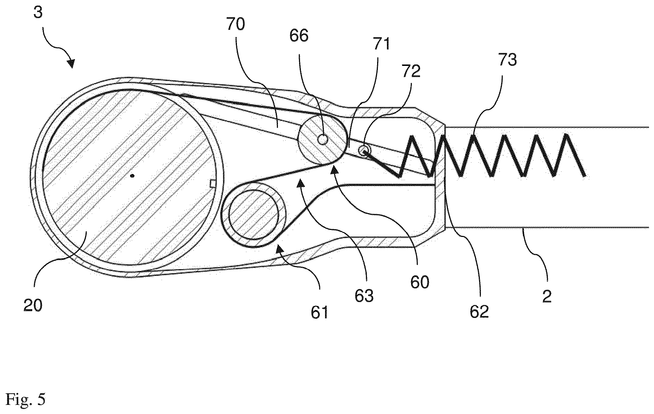

Die in den Figuren 4a bis 4c angedeutete Verschiebung der Achse 66 der Umlenkrolle 65 erfolgt entlang einer linearen Führung 70, die in Figur 5 schematisch dargestellt ist. Die Drehachse 66 ist mit einem Ausleger 71 verbunden, der in der Führung 70 verschiebbar geführt ist. Der Ausleger 71 trägt einen Stift 72, an dem eine in Fig. 5 angedeutete Feder 73 ansetzt. Die Darstellung der Fig. 5 ist dabei nur symbolisch zu verstehen; die Feder 73 ist ebenfalls innerhalb des hermetisch dichten Gehäuses 4 angeordnet und greift beispielsweise auf einer Innenseite des Gehäuses 4 an, so dass die Drehachse 66 zum Spannen des Kabels 62 belastet wird.

Das Kabel 62 wird, ausgehend von dem Anschlusspunkt 67, in einer ersten freien Strecke 62.1 bis zur Umlenkrolle 65, von dieser in einer zweiten freien Strecke 62.2 zum Umlenkzylinder 64 und von dort festliegend weitergeführt. Bei einer Drehung der Optikeinheit 20 im Gegenuhrzeigersinn wird das Kabel 62 auf die Umfangsfläche 68 aufgewickelt, wobei die Umlenkrolle 65 folgt, so dass die entsprechende Kabellänge bereitgestellt wird. Bei einer Drehung der Optikeinheit 20 im Uhrzeigersinn wird das Kabel 62 abgewickelt, wobei die Umlenkrolle 65 durch die Feder 73 zurückgezogen wird, so dass die abgewickelte Kabellänge aufgenommen wird. Durch die Federkraft der Feder 73 wird das Kabel 62 stets unter Spannung gehalten und somit sicher über die Umlenkungen 60, 61 geführt. Eine Krümmung des Kabels 62 mit einem unzulässig geringen Krümmungsradius und somit ein Knicken des Kabels 62 kann hierdurch sicher vermieden werden. Nach dem Durchtritt durch das Gehäuse 4 verläuft das Kabel 62 innerhalb des Schafts 2 des Exoskops bis in den Handgriff 50 und ist dort an den Anschluss 16 geführt, wo eine externe Versorgungs-, Auswertungs- und Darstellungseinrichtung anschließbar ist (s. Fig. 3). Der Durchtritt des Kabels 62 durch das Gehäuse 4, der keiner Bewegung des Kabels 62 ausgesetzt ist und daher auf einfache Weise hermetisch dicht ausgeführt werden kann, ist in den Figuren nicht dargestellt.

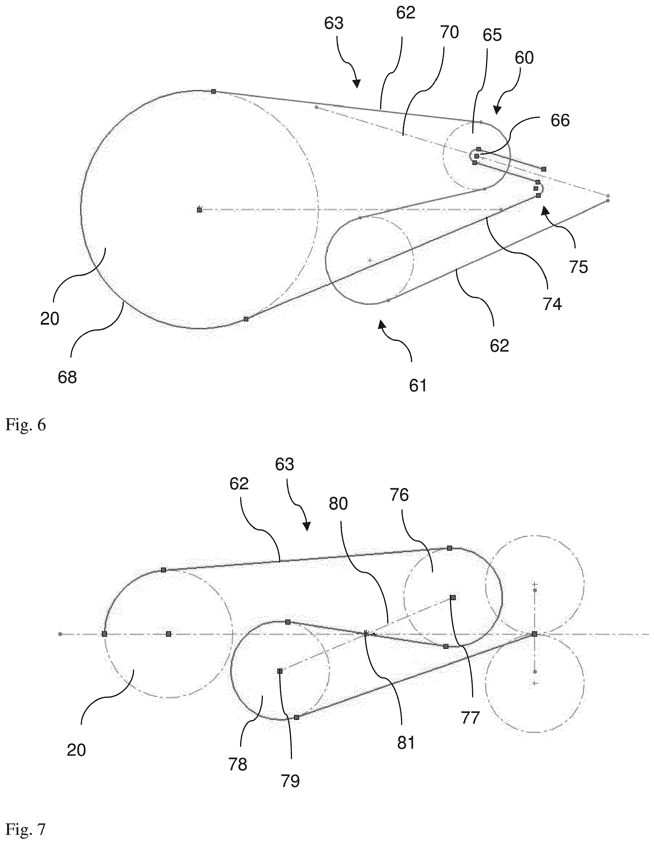

Eine zweite Ausführungsform eines Kabelreservoirs ist in Fig. 6 in schematischer Darstellung gezeigt. Das Kabel 62 wird, wie zum ersten Ausführungsbeispiel erläutert, ausgehend von der Optikeinheit 20 über eine erste Umlenkung 60 mit einer um eine Drehachse 66 drehbaren Rolle 65 und eine feststehende zweite Umlenkung 61 geführt, wobei die drehbare Rolle 65 linear verschiebbar geführt und zum Spannen des Kabels 62 sowie zum Nachführen der Rolle 65 durch eine Feder belastet ist. In einer darüber gelagerten Schicht ist ein flexibles Element, beispielsweise ein elastisch biegsamer Draht 74, der in umgekehrter Richtung auf die Umfangsfläche 68 der Optikeinheit 20 aufwickelbar bzw. von dieser abwickelbar ist, ebenfalls über zwei Umlenkungen geführt. Eine erste Umlenkung 75 des Drahts 74 ist feststehend ausgeführt, während eine zweite Umlenkung über eine drehbare Rolle erfolgt, die auf der Drehachse 66 der ersten Umlenkung 60 des Kabels drehbar gelagert ist; die Krümmungsradien der Umlenkungen des Drahts 74 können aufgrund der elastischen Eigenschaften des Drahts 74 anders, insbesondere kleiner als die der Umlenkungen des Kabels 62 sein. Der Draht 74 ist derart um die zweite Umlenkung geführt, dass der dadurch gebildete Bogen des Drahts 74 eine entgegengesetzte Richtung zu dem von der ersten Umlenkung 60 des Kabels 62 gebildeten Bogen aufweist. Der Draht 74 wird somit durch die an der Drehachse 66 ansetzende Feder ebenfalls unter Spannung gehalten, wodurch eine im Wesentlichen gleich große Kraft auf den Draht 74 ausgeübt wird wie auf das Kabel 62. Da das Kabel 62 und der Draht 74 in entgegengesetzter Richtung an der Optikeinheit 20 ansetzen, wird das vom Kabel 62 auf die Optikeinheit 20 ausgeübte Drehmoment durch den Draht 74 kompensiert.

Eine dritte Ausführungsform eines Kabelreservoirs ist schematisch in Fig. 7 dargestellt. Hierbei wird das Kabel 62, ausgehend von der Optikeinheit 20, über eine erste drehbare Rolle 76 mit einer Drehachse 77 und eine zweite drehbare Rolle 78 mit einer Drehachse 79 geführt. Die erste und die zweite drehbare Rolle 76, 78 sind mit ihren Drehachsen 77, 79 in einem Rahmen gelagert, der als zweiarmiger Hebel 80 mit einer Drehachse 81 ausgebildet ist. Die Drehachsen 77, 79 der Rollen 76, 78 und die Drehachse 81 des Hebels 80 stehen im Wesentlichen parallel zueinander. Der Hebel 80 ist durch eine nicht dargestellte Feder im Uhrzeigersinn vorgespannt. Wird das Kabel, ausgehend von der in Fig. 7 gezeigten Situation, durch eine Drehung der Optikeinheit 20 im Gegenuhrzeigersinn weiter auf die Umfangsfläche 68 aufgewickelt, so rotiert der Hebel 80 ebenfalls im Gegenuhrzeigersinn, wodurch die im Kabelreservoir 63 aufgenommene Länge des Kabels 62 verkürzt und ein Kabelabschnitt zum Aufwickeln auf die Umfangsfläche 68 der Optikeinheit 20 zur Verfügung gestellt wird. Dementsprechend wird bei einer Drehung der Optikeinheit 20 im Uhrzeigersinn der abgewickelte Kabelabschnitt durch eine Drehung des Hebels im Uhrzeigersinn im Kabelreservoir 63 aufgenommen. Wenn in einer entlang der Drehachse 81 über der in Fig. 7 gezeigten Anordnung gelagerten Schicht ein weiteres flexibles Element, das ein weiterer Teil des Kabels oder beispielsweise ein Draht sein kann, über eine gegengleiche Anordnung geführt wird, so wirkt ebenfalls kein Drehmoment auf die Optikeinheit.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste:

- 1: Exoskop
- 2: Schaft
- 3: Kopfteil
- 4: Gehäuse
- 4.1: Gehäuseunterteil
- 4.2: Gehäuseoberteil
- 5: Bodenfläche
- 6: Beobachtungsfenster
- 7: Beleuchtungsfenster
- 8: Deckelfläche
- 9: Drehkappe
- 10: Umfangsseite
- 11: Griffmulde
- 12: Oberseite
- 13: Drehgriff
- 14: Markierung
- 15: Handgriff
- 16: Anschluss
- 17: Gleitlager
- 18: Drehachse
- 19: Beleuchtungseinheit
- 20: Optikeinheit
- 21: Blickrichtung
- 22: Bildaufnehmer
- 25: Magnet
- 27: Magnetträger
- 28: Anschlag
- 32: Magnet
- 38: Magnetträger
- 39: Anschlag
- 40: Wellensicherungsring
- 41: Gleitdichtring
- 42: Gleitdichtring
- 50: Wärmeleitblech
- 51: Heatpipe
- 60: Umlenkung
- 61: Umlenkung
- 62: Kabel
- 62.1: Freie Strecke
- 62.2: Freie Strecke
- 63: Kabelreservoir
- 64: Zylinder
- 65: Rolle
- 66: Drehachse
- 67: Anschlusspunkt
- 68: Umfangsfläche
- 70: Führung
- 71: Ausleger
- 72: Stift
- 73: Feder
- 74: Draht
- 75: Umlenkung
- 76: Rolle
- 77: Drehachse
- 78: Rolle
- 79: Drehachse
- 80: Hebel
- 81: Drehachse

## Patentansprüche

1. Endoskop oder Exoskop mit einem Schaft (2) sowie mit mindestens einem Objektiv und mindestens einem elektronischen Bildaufnehmer zum Aufnehmen eines Bilds eines Objektfelds, wobei das mindestens eine Objektiv und der mindestens eine elektronische Bildaufnehmer in einem distalen Endbereich des Endoskops oder Exoskops (1) angeordnet sind, sowie mit mindestens einem innerhalb des Endoskops oder Exoskops (1) geführten Kabel (62) zur Versorgung und/oder Bildübertragung des elektronischen Bildaufnehmers, wobei der elektronische Bildaufnehmer von einer Optikeinheit (20) umfasst ist, die um eine erste Drehachse (18) drehbar gelagert ist, und an der das Kabel (62) angeschlossen und auf einer Umfangsfläche (68) der Optikeinheit (20) aufwickelbar ist, und wobei der distale Endbereich des Endoskops oder Exoskops (1) einen Hohlraum aufweist, in dem ein Kabelreservoir (63) zum Bereitstellen und Aufnehmen eines bei einer Drehung der Optikeinheit (20) auf- bzw. abgewickelten Kabelabschnitts des Kabels (62) aufgenommen ist, und wobei das Kabel (62) im Kabelreservoir (63) mindestens einen Bogen bildet, **dadurch gekennzeichnet, dass** die erste Drehachse (18) identisch mit oder parallel zu einer Blickrichtung (21) des Endoskops oder Exoskops ist, und dass das Kabel (62) im Bereich des mindestens einen Bogens durch mindestens eine in dem Hohlraum angeordnete Rolle (65, 76, 78) geführt ist.

2. Endoskop oder Exoskop nach Anspruch 1, **dadurch gekennzeichnet, dass** der Endbereich des Endoskops oder Exoskops (1) ein hermetisch dichtes Gehäuse (4) aufweist und dass die Optikeinheit (20) und das Kabelreservoir (63) in dem hermetisch dichten Gehäuse (4) aufgenommen sind.

3. Endoskop oder Exoskop nach Anspruch 1 oder 2, **dadurch gekennzeichnet, dass** die erste Drehachse (18) im Wesentlichen senkrecht zu einer Längsachse des Schafts (2) gerichtet ist.

4. Endoskop oder Exoskop nach Anspruch 3, **dadurch gekennzeichnet, dass** das Kabel (62) in einer im Wesentlichen tangentialen Richtung, die im Wesentlichen parallel zu der Längsachse des Schafts (2) gerichtet ist, auf die Umfangsfläche (68) der Optikeinheit (20) auf- bzw. von dieser abwickelbar ist.

5. Endoskop oder Exoskop nach einem der vorhergehenden Ansprüche, **dadurch gekennzeichnet, dass** das Kabel (62) in einem vollständig abgewickelten Zustand in Richtung der Längsachse des Schafts (2) betrachtet seitlich an der Optikeinheit (20) angeschlossen ist.

6. Endoskop oder Exoskop nach einem der vorhergehenden Ansprüche, **dadurch gekennzeichnet, dass** der den mindestens einen Bogen bildende Kabelabschnitt frei in den Hohlraum hinein gelegt ist.

7. Endoskop oder Exoskop nach einem der vorhergehenden Ansprüche, **dadurch gekennzeichnet, dass** der mindestens eine Bogen beim Auf- und Abwickeln des Kabels (62) seine Krümmungsrichtung beibehält.

8. Endoskop oder Exoskop nach einem der vorhergehenden Ansprüche, **dadurch gekennzeichnet, dass** die mindestens eine Rolle durch eine Beleuchtungseinheit (19) gebildet wird.

9. Endoskop oder Exoskop nach einem der vorhergehenden Ansprüche, **dadurch gekennzeichnet, dass** die mindestens eine Rolle (65) verschiebbar gelagert ist.

10. Endoskop oder Exoskop nach einem der vorhergehenden Ansprüche, **dadurch gekennzeichnet, dass** die mindestens eine Rolle (65) federbelastet verschiebbar gelagert ist.

11. Endoskop oder Exoskop nach einem der vorhergehenden Ansprüche, **dadurch gekennzeichnet, dass** der Kabelabschnitt durch mindestens zwei in dem Hohlraum angeordnete Rollen (76, 78) geführt ist, die auf einem zweiarmigen schwenkbaren Hebel (80) gelagert sind.

12. Endoskop oder Exoskop nach einem der vorhergehenden Ansprüche, **dadurch gekennzeichnet, dass** das Kabelreservoir (63) zwei in Richtung der ersten Drehachse (18) übereinanderliegende Schichten umfasst, wobei in einer ersten Schicht zumindest ein Teil des Kabels (62) und in einer zweiten Schicht ein weiterer Teil des Kabels (62) oder ein flexibles Element aufgenommen sind, wobei der in der zweiten Schicht aufgenommene Teil des Kabels (62) bzw. das in der zweiten Schicht aufgenommene flexible Element im Verhältnis zu dem in der ersten Schicht aufgenommenen Teil des Kabels (62) in entgegengesetzter Richtung auf der Umfangsfläche (68) der Optikeinheit (20) auf- bzw. abwickelbar ist und im Kabelreservoir (63) gegenläufig geführt ist.

## Claims

1. Endoscope or exoscope with a shank (2) and with at least one objective and at least one electronic image recorder for recording an image of an object field, wherein the at least one objective and the at least one electronic image recorder are arranged in a distal end area of the endoscope or exoscope (1), and with at least one cable (62), guided inside the endoscope or exoscope (1), for supplying power to and/or transmitting images from the electronic image recorder, wherein the electronic image recorder is part of an optical unit (20) which is mounted rotatably about a first rotation axis (18) and to which the cable (62) is attached and can be wound on a circumferential surface (68) of the optical unit (20), and wherein the distal end area of the endoscope or exoscope (1) has a cavity in which a cable store (63) is received for providing and receiving a cable portion of the cable (62) that is wound or unwound during a rotation of the optical unit (20), and wherein the cable (62) in the cable store (63) forms at least one arc, **characterized in that** the first rotation axis (18) is identical to or parallel to a viewing direction (21) of the endoscope or exoscope, and **in that** the cable (62), in the area of the at least one arc, is guided by at least one reel (65, 76, 78) arranged in the cavity.

2. Endoscope or exoscope according to Claim 1, **characterized in that** the end area of the endoscope or exoscope (1) has a hermetically sealed housing (4), and **in that** the optical unit (20) and the cable store (63) are received in the hermetically sealed housing (4).

3. Endoscope or exoscope according to Claim 1 or 2, **characterized in that** the first rotation axis (18) is oriented substantially perpendicularly with respect to a longitudinal axis of the shank (2).

4. Endoscope or exoscope according to Claim 3, **characterized in that** the cable (62) can be wound onto and unwound from the circumferential surface (68) of the optical unit (20) in a substantially tangential direction which is oriented substantially parallel to the longitudinal axis of the shank (2).

5. Endoscope or exoscope according to one of the preceding claims, **characterized in that**, in a completely unwound state, the cable (62) is connected laterally to the optical unit (20) when viewed in the direction of the longitudinal axis of the shank (2).

6. Endoscope or exoscope according to one of the preceding claims, **characterized in that** the cable portion forming the at least one arc is placed freely into the cavity.

7. Endoscope or exoscope according to one of the preceding claims, **characterized in that** the at least one arc maintains its direction of curvature during the winding and unwinding of the cable (62).

8. Endoscope or exoscope according to one of the preceding claims, **characterized in that** the at least one reel is formed by an illumination unit (19).

9. Endoscope or exoscope according to one of the preceding claims, **characterized in that** the at least one reel (65) is mounted in a displaceable manner.

10. Endoscope or exoscope according to one of the preceding claims, **characterized in that** the at least one reel (65) is mounted in a displaceable manner under spring loading.

11. Endoscope or exoscope according to one of the preceding claims, **characterized in that** the cable portion is guided by at least two reels (76, 78) arranged in the cavity, which reels (76, 78) are mounted on a two-armed pivotable lever (80).

12. Endoscope or exoscope according to one of the preceding claims, **characterized in that** the cable store (63) comprises two layers lying one above the other in the direction of the first rotation axis (18), wherein at least one part of the cable (62) is received in a first layer and a further part of the cable (62) or a flexible element is received in a second layer, wherein the part of the cable (62) received in the second layer or the flexible element received in the second layer can be wound or unwound in the opposite direction on the circumferential surface (68) of the optical unit (20), compared to the part of the cable (62) received in the first layer, and is guided in the opposite direction in the cable store (63).

## Revendications

1. Endoscope ou exoscope avec une tige (2), ainsi qu'avec au moins un objectif et au moins un dispositif d'imagerie électronique en vue de la prise d'une image d'un champ d'objectif, selon lequel l'au moins un objectif et l'au moins un dispositif d'imagerie électronique sont disposés dans une zone de l'extrémité distale de l'endoscope ou de l'exoscope (1), ainsi qu'avec au moins un câble (62) qui est guidé à l'intérieur de l'endoscope ou de l'exoscope (1) en vue de l'alimentation et/ou de la transmission des images du dispositif d'imagerie électronique, selon lequel le dispositif d'imagerie électronique est entouré par une unité optique (20), laquelle est positionnée de façon à pouvoir pivoter autour d'un premier axe de rotation (18) ;
et
laquelle est raccordée au câble (62) et peut être enroulée sur une surface circonférentielle (68) de l'unité optique (20), et selon lequel la zone de l'extrémité distale de l'endoscope ou de l'exoscope (1) présente une cavité, dans laquelle un réservoir de câble (63) est mis en place en vue de la mise à disposition et de la réception d'une section de câble du câble (62), laquelle est enroulée ou déroulée quand une rotation de l'unité optique (20) est réalisée, et selon lequel le câble (62) forme au moins un arc dans le réservoir de câble (63), **caractérisé en ce que** le premier axe de rotation (18) est identique ou parallèle à une direction de visée (21) de l'endoscope ou de l'exoscope et **caractérisé en ce que** le câble (62) est guidé, dans la zone de l'au moins un arc, par l'intermédiaire d'au moins un galet (65, 76, 78), lequel est disposé dans la cavité.

2. Endoscope ou exoscope selon la revendication 1, **caractérisé en ce que** la zone de l'extrémité de l'endoscope ou de l'exoscope (1) présente un boîtier (4) hermétiquement étanche et **caractérisé en ce que** l'unité optique (20) et le réservoir de câble (63) sont logés dans le boîtier (4) hermétiquement étanche.

3. Endoscope ou exoscope selon la revendication 1 ou 2, **caractérisé en ce que** le premier axe de rotation (18) est, pour l'essentiel, orienté de manière perpendiculaire à un axe longitudinal de la tige (2).

4. Endoscope ou exoscope selon la revendication 3, **caractérisé en ce que** le câble (62) peut, pour l'essentiel, être enroulé dans une direction tangentielle sur la surface circonférentielle (68) de l'unité optique (20), respectivement peut être déroulé dans une direction tangentielle de la surface circonférentielle de l'unité optique, laquelle direction tangentielle est orientée, pour l'essentiel, parallèlement à l'axe longitudinal de la tige (2).

5. Endoscope ou exoscope selon l'une des revendications précédentes, **caractérisé en ce que** le câble (62) est raccordé de manière latérale au niveau de l'unité optique (20), considéré dans un état déroulé en intégralité dans la direction de l'axe longitudinal de la tige (2).

6. Endoscope ou exoscope selon l'une des revendications précédentes, **caractérisé en ce que** la section de câble, laquelle forme l'au moins un arc, est mise en place de manière lâche à l'intérieur de la cavité.

7. Endoscope ou exoscope selon l'une des revendications précédentes, **caractérisé en ce que** l'au moins un arc conserve sa direction de courbure lors de l'enroulement et du déroulement du câble (62).

8. Endoscope ou exoscope selon l'une des revendications précédentes, **caractérisé en ce que** l'au moins un galet est formé par une unité d'éclairage (19).

9. Endoscope ou exoscope selon l'une des revendications précédentes, **caractérisé en ce que** l'au moins un galet (65) est positionné de manière coulissante.

10. Endoscope ou exoscope selon l'une des revendications précédentes, **caractérisé en ce que** l'au moins un galet (65) est positionné de manière coulissante en étant monté sur un ressort.

11. Endoscope ou exoscope selon l'une des revendications précédentes, **caractérisé en ce que** la section de câble est guidée par au moins deux galets (76, 78) qui sont disposés dans la cavité et qui sont positionnés sur un levier (80) à deux bras pouvant pivoter.

12. Endoscope ou exoscope selon l'une des revendications précédentes, **caractérisé en ce que** le réservoir de câble (63) comprend deux couches superposées dans la direction du premier axe de rotation (18), selon lequel tout au moins une partie du câble (62) est mise en place dans une première couche et selon lequel une autre partie du câble (62) ou un élément flexible est mis(e) en place dans une deuxième couche, selon lequel la partie du câble (62) mise en place dans la deuxième couche, respectivement l'élément flexible mis en place dans la deuxième couche, peut être enroulé (e) ou peut être déroulé(e) sur la surface circonférentielle (68) de l'unité optique (20) dans une direction opposée par rapport à la partie du câble (62) mise en place dans la première couche, et est guidé (e) dans la direction inverse dans le réservoir de câble (63).
